# EUROPÄISCHE PATENTSCHRIFT

(11) **EP 3 407 842 B1**
(45) Veröffentlichungstag und Bekanntmachung des Hinweises auf die Patenterteilung: **27.05.2020**
(21) Anmeldenummer: 17702826.3
(22) Anmeldetag: 27.01.2017
(51) Int. Cl.: A61F 5/02

(54) **ZUGGURT-REKLINATIONSORTHESE**
RECLINER-TYPE ORTHOSIS COMPRISING TIGHTENING STRAPS
ORTHÈSE DE RÉCLINAISON À SANGLES DE TRACTION

(30) Priorität: 28.01.2016 DE 102016201270
(43) Veröffentlichungstag der Anmeldung: 05.12.2018
(73) Patentinhaber: Bauerfeind AG, 07937 Zeulenroda-Triebes (DE)
(72) Erfinder: STIER, Gerald, 07957 Langenwetzendorf (DE); HARMS, Jürgen, 76227 Karlsruhe (DE)
(74) Vertreter: Schwahn, Hartmut
(86) Internationale Anmeldenummer: PCT/EP2017/051803
(87) Internationale Veröffentlichungsnummer: WO 2017/129762

(56) Entgegenhaltungen:
- EP-A1- 2 070 495
- CN-U- 201 631 437
- DE-A1- 10 329 454
- GB-A- 2 159 058
- US-A- 945 359
- US-A1- 2008 228 121
- US-A1- 2010 318 010
- US-A1- 2012 245 501

## Beschreibung

Die Erfindung betrifft eine Rückenorthese zur Behandlung und Aufrichtung einer verkrümmten Wirbelsäule beziehungsweise zur Behandlung und Korrektur von Haltungsfehlern besonders im oberen Rückenbereich durch Repositionierung der Schultern. Es wird eine neuartige Reklinationsorthese mit spezieller Gurtführung bereitgestellt, die in ihrer Funktion höchst wirksam ist und leicht anlegbar und anpassbar ist.

Reklinationsorthesen, auch Aufrichtungs-Orthesen genannt, bestehen im Wesentlichen aus einem starren Versteifungselement, das entlang der Wirbelsäule verläuft ("Reklinator"), das über Gurte und Bandagen, die den Rumpf des Patienten unter Spannung umgreifen, an die Wirbelsäule gepresst wird, um dort die Stützwirkung zu vermitteln. Bekannte Reklinationsorthesen umgreifen mit zwei Schultergurten die Schulterpartien und ziehen beim Tragen die Schultern nach hinten. Durch Zusammenwirken der Schultergurte, dem Reklinator und der rumpfumgreifenden Bandage wird die Wirbelsäule des Patienten mechanisch stabilisiert und vor allem im Bereich der Schulter, der oberen Brustwirbelsäule aufgerichtet. Je stärker die Schultergurte gespannt werden, desto stärker werden die Schultern nach hinten gezogen, wodurch der Oberkörper aufgerichtet wird. Schwerpunkt der Stützwirkung liegt bei bekannten Reklinationsorthesen im Bereich der oberen Brustwirbel bis hin zu den Halswirbeln. Häufig findet sich aber vor allem bei degenerativen Erkrankungen der Wirbelsäule, insbesondere bei Osteoporose, einer Schwächung aller Wirbelkörper, und es wird erforderlich, die Wirbelsäule auch im Bereich der unteren Brustwirbel, der Lendenwirbel und/oder des Kreuzbeins mechanisch zu stützen und gegebenenfalls mit aufzurichten. Dies können bekannte Reklinationsorthesen noch nicht ausreichend leisten.

Aus der US 2012/245501 A1, US 2008/228121 A1, US 2010/318010 A1, EP 2 070 495 A1, GB 2 159 058 A, DE 103 29 454 A1 und US 945 359 A sind Rückenorthesen mit Beckengurten, Schultergurten und gegebenenfalls starren Rückenschienen bekannt. CN201631437U zeigt eine Rückenorthese mit Schultergurten die sich in einem dorsalen ventralen Kreuzpunkt und in lateralen thorakalen Kreuzpunkten kreuzen.

Nachteilig bei bekannten Reklinationsorthesen ist außerdem der komplexe Aufbau und die starre Ausgestaltung der Bandagen und Zuggurte, welche rein auf die Erzielung einer spezifischen Stützwirkung hin ausgebildet sind, jedoch eine individuelle Anpassung an die spezifische Indikationslage, an das Therapieerfordernis und/oder an die anatomischen Gegebenheiten nicht genügend zulassen. Besonders bei Bewegung des Patienten kommt es je nach Bewegungsphase zu einem Ausbleiben der Stützwirkung, weil bekannte Reklinationsorthesen der Bewegung nicht ausreichend folgen können. Außerdem sind bekannte Reklinationsorthesen vom Patienten häufig schwer anzulegen, da die dann zur Aufrichtungs- und Stützwirkung erforderliche mechanische Kraft, besonders Zugkraft, Spannkraft, durch den Patienten selbst nicht ausreichend aufgebracht werden kann. Dies ist besonders erschwert bei älteren Patienten.

Der vorliegenden Erfindung lag das technische Problem zugrunde, eine verbesserte Reklinationsorthese bereitzustellen, die eine Stützwirkung über die gesamte Länge der Wirbelsäule bis hin zum Becken vermitteln kann, spezifisch anpassbar ist und vom Patienten selbst leicht und wirkeffektiv angelegt werden kann. Das technische Problem wird vollständig gelöst durch eine Reklinationsorthese gemäß den Ansprüchen, besonders also eine Reklinationsorthese mit einer starren, im angelegten Zustand entlang der gesamten Wirbelsäule verlaufenden Reklinatorschiene, einem insbesondere auf Beckenkamm und Becken aufsitzenden, um das Becken spannbaren Beckengurt sowie zwei solitäre, insbesondere freilaufende, spannbare Schultergurte. Bevorzugt besteht die erfindungsgemäße Reklinationsorthese ausschließlich aus diesen stützenden krafteinleitenden Elementen ohne weiteren Bandagen und Gurtelementen, abgesehen von unselbstständigen passiven Hilfsapplikationen wie Polsterungen, Pelotten und Gurtführungen und Verschlusssystemen.

Die Gurte der erfindungsgemäßen Reklinationsorthese weisen eine ganz besondere und neuartige Gurtführung um den Körper des Patienten herum mit zumindest drei konkreten Gurtkreuzungen auf. Erfindungsgemäß sind beide Gurte dabei jeweils ausgehend von einem caudalen, besonders tief gelegenen Abschnitt der Reklinatorschiene. Sie sind von dort jeweils nach cranial, "oben" verlaufend, an der ipsilateralen Körperflanke am Brustkorb des Patienten, das heißt unter der Achsel, entlang, über die jeweilige ipsilaterale vordere Schulter, wo sie jeweils Schultergurtabschnitte bilden, dann zurück nach dorsal an einem cranialen Abschnitt dieser Reklinatorschiene vorbei, wo sie insbesondere frei in Führungen geführt sind, nach caudal, "unten". Die Gurte sind dabei in einem zentralen dorsalen cranialen Kreuzungspunkt kreuzend und weiter caudal nach jeweils kontralateral verlaufend, und zwar an der kontralateralen Körperflanke am Brustkorb des Patienten, das heißt unter der Achsel, entlang nach ventral ziehend. Der kontralateral laufende Gurt ist dabei in einem lateralen thorakalen Kreuzungspunkt, der sich bevorzugt zwischen Achsel und Beckenkamm befindet, mit dem jeweils ipsilateralen aufsteigend verlaufenden Gurtpartner kreuzend. Schließlich sind die kontralateral laufenden Gurte jeweils nach der lateralen thorakalen Kreuzung ventral in Gurtenden auslaufend, die unter Spannkraft unmittelbar an dem ventralen Abschnitt des Beckengurts der Orthese festlegbar sind.

Die erfindungsgemäße Reklinationsorthese wird also zunächst in der Art eines Rucksacks mit zwei solitären Schultergurten angelegt und am Becken mittels Beckengurt, welcher mit der Reklinatorschiene fest verbunden ist, an den Körper gespannt. Die freien Gurtenden der der am caudalen Abschnitt der Reklinatorschiene festgelegten einzelnen Gurte sind dabei einzeln zieh- und spannbar, wobei die Zugkraft sich durch die insbesondere freilaufenden Gurte auf wichtige Druckeinleitungspunkte der Orthese, nämlich: vordere Schulter/Schlüsselbein - cranialer Abschnitt der Reklinatorschiene - caudaler Abschnitt der Reklinatorschiene - Beckengurt automatisch verteilen kann und so jeweils ein in sich geschlossener Kraftschlusskreis gebildet werden kann. Die Gurte bilden im Bereich der Schulter jeweils Schultergurtabschnitte, an denen die hauptsächliche Krafteinleitung der Gurte in den Körper erfolgt. Die Schultern können dadurch therapiegerecht nach hinten und die Reklinatorschiene gleichzeitig an die Wirbelsäule gezogen und so obere Brust- und Halswirbelsäule aufgerichtet werden.

Durch den Beckengurt, der hier erfindungsgemäß insbesondere stets unterhalb des Bauches des Patienten verläuft, insbesondere in der Höhe des Ileosakralgelenks, und zumindest dort mit dem cranialen Abschnitt der Reklinatorschiene fest verbunden ist, wird die Stützwirkung des Reklinators auch auf die untere Brustwirbelsäule, die Lendenwirbelsäule und das Kreuzbein erstreckt. Durch die insbesondere freie Gurtführung der beiden körperumlaufenden Schultergurte, die jeweils nur an dem caudalen Abschnitt der Reklinatorschiene einerseits und an dem ventralen Abschnitt des Beckengurts andererseits festgelegt oder festlegbar sind, wird vorteilhafterweise eine neue Bewegungsfreiheit des Patienten ermöglicht. Gleichzeitig sitzt die Orthese positionsstabil am Körper und passt sich durch den geschlossenen Kraftschlusskreis stets dynamisch an die Bewegungsphasen an.

Besonders vorteilhaft wird eine dynamische Kraftverteilung an den Druckeinleitungspunkten, je nach Bewegungszustand und Haltung des Patienten, realisiert, welche die Stützwirkung und Reklination in jeder Bewegungsphase sichert und gleichzeitig den Tragekomfort für den Patienten erhöht. Diese dynamische Kraftverteilung in der Bewegung ermöglicht dem Patienten, aktiv eine aufrechte Haltung einzunehmen und die Stützwirkung seiner Reklinationsorthese durch aktive Muskeltätigkeit, bewusst und teilweise reflexiv, zu unterstützen. Besonders sind dabei durch die neuartige Gurtführung weitere insbesondere solitäre Druckeinleitungspunkte im Bereich der Beckenfassung, im Bereich des vorderen Beckenkamms und insbesondere auch an den beiden thorakalen lateralen Gurtkreuzungen an den Brustkorbseiten gebildet, die auch zu einer reflektorischen Aktivierung der Haltemuskulatur beitragen können. Der befürchtete Nachteil bekannter statischer Stützorthesen, nämlich die Schwächung der Haltemuskeln, wird bei der erfindungsgemäßen Reklinationsorthese mit dynamischer Kraftverteilung und Druckeinleitung nicht nur vermieden; es wird vielmehr ein Trainingseffekt für die Haltemuskulatur erzielt. Dies ist insbesondere in der Rehabilitation, beispielsweise nach Unfällen oder Wirbelsäulenverletzungen anderer Art, beispielsweise nach Operation, von großem Nutzen. Die erfindungsgemäße Reklinationsorthese erreicht diese Vorteile bei gleichzeitig einfachem mechanischen Aufbau und geringer Anzahl an spezifischen Bauelementen.

In einer bevorzugten Ausgestaltung sind die Verankerungspunkte der beiden Schultergurte an der Reklinatorschiene, im Bereich des caudalen Abschnitts der Reklinatorschiene frei wählbar. Besonders sind dort mehrere Verankerungspunkte vorgesehen, wo Ankerösen, durch welche jeweils die Gurte geführt und daran fixiert sind, in der Position wählbar festlegbar sind. Obwohl ein Erfindungsmerkmal einen tiefen Ansatzpunkt der beiden körperumlaufenden Schultergurte, bevorzugt im Bereich des Ileosakral-Gelenks, am dorsalen Beckenabschnitt des Patienten vorsieht, wobei die Gurte an dem dort positionierbaren caudalen Abschnitt der Reklinatorschiene oder dem Beckengurt dort festgelegt oder festlegbar sind, kann die Höhe dieses caudalen Gurtansatzes entlang der Reklinatorschiene variiert werden. Dies dient spezifisch der Anpassung der Therapiewirkung der Reklinationsorthese einerseits, alternativ oder zusätzlich der Anpassung an die Körpergröße und die anatomischen Verhältnisse, besonders Rumpflänge, Beckenumfang, andererseits, beispielsweise bei Patienten mit aufgrund degenerativer Erkrankungen bereits stark verkürzten Wirbelkörpern. So kann der Orthopädietechniker beim Anpassen der Orthese diese Parameter beim Patienten überprüfen und den jeweils günstigen Ansatzpunkt der beiden Gurte in caudalen Abschnitt der Reklinatorschiene wählen, welcher die für die jeweilige Therapie zweckmäßige Druckeinleitung und Kraftverteilung ermöglicht. Die durch die Ankerösen geführten freien Gurtenden sind bevorzugt variabel an sich selbst festlegbar, beispielsweise durch Klettverbindungen oder durch Verknopfungen, Verhakungen. Damit kann die Gesamtlänge der Gurte einfach angepasst werden. In einer spezifischen Ausführung sind die Ankerösen als klemmende Doppelösen ausgestaltet, die ein stufenloses Verstellen der Gurtlänge mit automatischer Verklemmung an den Ankerösen ermöglichen.

In bevorzugter Ausgestaltung sind am oberen, das heißt cranialen Abschnitt der Reklinatorschiene Gurtführungen, das heißt Führungselemente, für die Gurte, durch welche die Gurte insbesondere frei gleiten können, besonders Führungsösen vorgesehen. In einer bevorzugten Variante sind diese Führungen beziehungsweise Führungsösen positionsvariabel an dem cranialen Abschnitt der Reklinatorschiene festgelegt. In einer ersten Variante sind an oder in der Reklinatorschiene mehrere zueinander beabstandete Ausnehmungen vorgesehen, durch welche die Gurte gleiten können. In einer alternativen Variante sind dort separate Führungsösen vorgesehen, die auf die Reklinatorschiene positionsvariabel festgelegt werden können, insbesondere durch Verklettung, Verknopfung oder Verschraubung. Diese Führungselemente dienen erfindungsgemäß als Führungs- und gleichzeitig Umlenkpunkte der Gurte am cranialen Abschnitt der Reklinatorschiene. Sind die Gurte gespannt, dienen die Führungen dort zur Krafteinleitung in die Reklinatorschiene. Durch Variation der Position der Führungselemente an der Reklinatorschiene kann die Krafteinleitung an das Therapieziel und/oder an die Größenverhältnisse am Patienten angepasst werden. In einer bevorzugten Variante sind die Führungselemente vollständig von der Reklinatorschiene abrüstbar. So kann die gesamte Orthesenkonstruktion leicht zerlegt und insbesondere die Reklinatorschiene entnommen werden, ohne dass die Schultergurte komplett von der Schiene abgerüstet werden müssen. Auch kann so das Anlegen der Orthese erleichtert werden.

Besonderes Merkmal der erfindungsgemäßen Reklinationsorthese ist die spezifische Gurtführung der beiden solitären, insbesondere freilaufenden Schultergurte, die jeweils von einem caudalen Abschnitt der Reklinatorschiene ausgehen und sich jeweils seitlich unter der Achse des Patienten über den Brustkorb in zwei lateralen thorakalen Kreuzungspunkten, seitlich am Körper des Patienten in der Höhe des Brustkorbs unter den Achseln, mit ihrem kontralateralen Gurtpartner im Wesentlichen in einem 90° Winkel kreuzen. Idealerweise kreuzen die Gurte etwa jeweils mittig auf einer zwischen Achselhöhle und Beckenkamm gedachten Linie, besonders bevorzugt sogar unterhalb der Mitte dieser Linie in Richtung Beckenkamm. In einer bevorzugten Variante davon werden die Gurte an diesen seitlichen Kreuzungspunkten in speziellen Kreuzungsführungen geführt, wobei spezifisch ein Kreuzungswinkel von etwa 90° voreingestellt ist. Diese Kreuzungsführungen sind in einer bevorzugten Ausgestaltung der erfindungsgemäßen Reklinationsorthese spezifisch ausgebildet, zumindest einen der Gurte, bevorzugt beide Gurte, in daran ausgebildeten Führungsösen oder entsprechenden Ausnehmungen frei gleiten zu lassen, sodass sich die Kreuzung der beiden Gurte am Kreuzungspunkt nach Anlegen der Reklinationsorthese und Spannen der beiden Gurte selbst "findet". Diese erfindungsgemäß bevorzugte "selbstfindende" laterale Kreuzung der Gurte im Bereich zwischen Achselhöhle und Beckenkamm erlaubt und/oder unterstützt die erfindungsgemäß erzielbare dynamische Druckverteilung an der Reklinationsorthese. Diese mechanische Kreuzungsführung erleichtert außerdem das Anlegen und Spannen der Reklinationsorthese an dem und vor allem durch den Patienten mittels der beiden freien und festlegbaren Gurtenden dieser erfindungsgemäß den Körper des Patienten umlaufenden und sich kreuzenden Schultergurte.

Diese Kreuzungsführungen können zusätzlich auf der zum Körper gewandten Seite mit einer Polsterung und spezifisch mit einer zusätzlich druckeinleitenden Pelotte versehen sein. Diese Pelotte unterstützt dabei bevorzugt die dynamische Druckverteilung an der angelegten Reklinationsorthese zum Zwecke der aktiven Haltungsverbesserung.

In einer alternativen Variante sind diese Kreuzungsführungen spezifisch ausgebildet, um zumindest einen der beiden, besonders bevorzugt alle beide geführten Gurte dort festzulegen. Es können dort entsprechende Gurtklemmen oder Rasten ausgebildet sein. Dadurch kann, je nach therapeutischem Ansatz und/oder anatomischen Verhältnissen das dynamische "Selbstfinden" des Kreuzungspunkts der beiden Gurte beeinflusst oder unterdrückt oder fixiert werden. Durch das Festlegen der Kreuzungsführung an zumindest einem der beiden dort kreuzenden Gurte kann die Kreuzungsführung selbst Scher- und Tangentialkräfte aufnehmen und, besonders in Verbindung mit einer Pelotte, lokal körperstützend wirken. Alternativ oder zusätzlich kann durch die an mindestens einem der Gurte festlegbare Kreuzungsführung ein zu starkes Verschieben des lateralen Kreuzungspunkts am Patienten, je nach Bewegungszustand, beispielsweise ein Verrutschen des Kreuzungspunkts zu weit nach oben in Richtung der Achselhöhlen, verhindert werden. In einer besonderen Ausgestaltung der Kreuzungsführungen sind diese so ausgestaltet, dass zwischen einem freien Durchlaufen der Gurte und einer lokalen Festlegung der Gurte wiederkehrend gewechselt werden kann. So kann beispielsweise der Orthopädietechniker beim Anpassen der Reklinationsorthese an den Patienten die entsprechende Funktion wählen. Besonders ist aber vorgesehen, dass die Gurte in den Kreuzungsführungen zunächst frei laufen, während die Reklinationsorthese am Patienten angelegt wird, die Schultergurte dann gespannt werden und im Funktionszustand, nachdem sich die lateralen Kreuzungspunkte beim Anlegen selbst "gefunden" haben, die Gurte nun an den Kreuzungsführungen festgelegt werden können, um ein Verschieben des einmal gefundenen Kreuzungspunkts beispielsweise bei exzessiver Bewegung im Rahmen sportlicher Aktivität oder physiotherapeutischer Übungen zu verhindern.

Der Beckengurt, mit welcher die Reklinatorschiene bevorzugt fest verbunden ist, ist zwar bevorzugt in an sich bekannter Weise ausgestaltet: zwei breite Bandagengurte erstrecken sich jeweils zu beiden Seiten der Reklinatorschiene und lassen sich um den Rumpf herum führen und schließen. Aber dieser Beckengurt ist spezifisch ausgebildet, dass er um den Beckenkamm und/oder das Becken geführt und unter Aufbringung von Spannung über den Unterbauch/Schambein des Patienten unter Spannung geschlossen werden kann. Bevorzugt sind dabei überlappende Bandagenenden, welche mittels Klettverbindung aneinander zugfest verklettet werden. Alternativ sind Verhakungen oder Verknopfungen zum Verbinden der beiden Bandagenenden. Erfindungsgemäß ist der Beckengurt der Orthese also sehr tief am Rumpf, und zwar am Beckenkamm und darunter, positioniert. Dieser tiefe Ansatz unterstützt die Reklinationswirkung der Orthese deutlich. Er ermöglicht den tiefen Ansatz der beiden Gurte an der Reklinatorschiene im Bereich des Ileosakral-Gelenks.

In bevorzugter Ausgestaltung sind die beiden Bandagenenden des Beckengurts in dem ventralen Abschnitt zusätzlich mit Mitteln versehen, welche dort ein Festlegen der beiden gespannten Schultergurte unter Spannung ermöglichen. Bevorzugt sind hier Klettverbindungselemente, beispielsweise Hakenelemente auf der körperzugewandten Seite der Gurtenden und Flauschelemente auf der gegenüberliegenden Oberseite. So können die Bandagenenden und die beiden Gurtenden der Schultergurte einfach übereinandergelegt und damit miteinander zugfest verbunden werden. Durch eine entsprechend große Klett-Flausch-Fläche im Bereich des ventralen Abschnitts des Beckengurts wird vorteilhafterweise eine variable Festlegung der beiden Gurtenden und damit die Variation der Zugspannung und damit der Krafteinleitung in die Reklinationsorthese ermöglicht. Der Patient kann somit leicht selbst die gewünschte Stützwirkung seiner Reklinationsorthese je nach Therapieziel und/oder Bewegungsablauf anpassen. Alternative Mittel zur Festlegung der Gurtenden im ventralen Bereich des Beckengurts sind Verknopfungen, Verhakungen.

In einer bevorzugten Variante sind die Gurtenden der beiden Schultergurte zusätzlich mit Eingriffsmöglichkeiten, bevorzugt in Form von Taschen oder Henkeln, versehen, welche ein leichtes Ergreifen und Spannen der Gurtenden für den Patienten ermöglichen. Dies ist insbesondere für ältere Patienten, die eine eingeschränkte Handbeweglichkeit aufweisen, von großem Vorteil.

In den hierin primär beschriebenen Ausgestaltungen der erfindungsgemäßen Reklinationsorthese sind die beiden erfindungsgemäßen Schultergurte/Spanngurte in Form von flachen, flexiblen und im Wesentlichen unelastischen Gurtbändern ausgebildet. Die Breite der Bänder beträgt dabei zweckmäßig von etwa 1 bis etwa 10 cm, bevorzugt von 2 bis 6 cm. Im Bereich der Hauptpunkte der Druckeinleitung, besonders im Bereich der vorderen Schulterabschnitte, im Bereich des Schlüsselbeins sind die Gurte bevorzugt zusätzlich mit an sich bekannten Polsterelementen oder Pelotten zur gezielten Druckeinleitung versehen. Bevorzugt sind diese am Gurt verschieblich und können so beim Anlegen der Orthese am Körper vorpositioniert werden, wobei beim anschließenden Spannen der Gurte sich diese Polsterungen nicht mehr am Körper verschieben, sondern vielmehr die Gurte in den Polsterungen frei durchlaufen können. In besonderen Varianten davon sind die Polsterungen jeweils an den Gurten festgelegt, insbesondere festgenäht, um das Anlegen der Reklinationsorthese, besonders bei bewegungseingeschränkten Patienten zu erleichtern. Bevorzugt befinden sich solche Gurtpolsterungen zumindest im Bereich der vorderen Schultern, des Schlüsselbeins. Zusätzlich ist bevorzugt eine Polsterung im Bereich des Beckenkamms vorgesehen, an der Stelle, wo die Gurte nach Durchlaufen der lateralen thorakalen Kreuzungen nach ventral zu den Gurtenden auslaufen. Durch die laterale Kreuzung und Festlegung der Gurtenden im ventralen Abschnitt des Beckengurts bildet sich so bevorzugt ein zusätzlicher Druckeinleitungspunkt zwischen lateralem Kreuzungspunkt und Ort der ventralen Festlegung der Gurtenden. Dieser Druckeinleitungspunkt liegt bei der erfindungsgemäßen Reklinationsorthese im Bereich des Beckenkamms; er unterstützt die Stützfunktion der erfindungsgemäßen Reklinationsorthese insbesondere im Bereich der unteren Brustwirbel, der Lendenwirbel und Sakralwirbel. Ohne an die Theorie gebunden sein zu wollen, unterstützt der zusätzliche Druckeinleitungspunkt im ventralen Bereich des Beckenkamms eine kraftschlüssige Verbindung der Reklinatorschiene mit dem Becken und dem caudalen Abschnitten der Wirbelsäule, besonders dem Ileosakral-Gelenk. Durch diese beckenseitige Krafteinleitung verbessert sich rückwirkend, aufgrund des Kraftschlusses über die laufenden Schultergurte, auch die Reklination im Bereich der oberen Brustwirbel und Halswirbel bei gleichzeitig vorteilhafter dynamischer Druckverteilung.

In einer alternativen Ausgestaltung sind die erfindungsgemäßen Schultergurte zusätzlich oder ausschließlich als Zugdrähte oder Zugschnüre ausgebildet, die an den entsprechenden Druckeinleitungspunkten mit Polsterungen und/oder Pelotten versehen sind. Diese Zugseile können beispielsweise in textilen Kanälen verdeckt laufen, die selbst als Polsterung dienen. Die Ausgestaltung der Gurte als Zugseile, die spezifisch in textilen Kanälen laufen, ermöglicht eine leichte und besonders luftige oder atmungsaktive Bauweise. Auch kann die Reklinationsorthese dann kleiner zusammengelegt und in kleineren Verkaufsverpackungen gelagert und angeboten werden.

Die Reklinatorschiene besteht in einer bevorzugten Ausgestaltung aus einem starren Reklinatorkern, der in einer Reklinatortasche, vorzugsweise eine Textiltasche, aufgenommen ist und dort fest gehalten wird. Die Tasche ist bevorzugt mit dem Beckengurt vernäht oder verschweißt. Die Tasche dient der Polsterung des starren Reklinatorkerns sowie zusätzlich bevorzugt zur Fixierung zusätzlicher spezifisch positionierbarer Polsterungen und/oder Pelotten entlang der Reklinatorschiene zur gezielten therapeutisch zweckmäßigen Druckeinleitung an der Wirbelsäule. Bevorzugt ist die Reklinatorschiene frei in diese Tasche eingelegt und aus dieser herausnehmbar, um vom Orthopädietechniker unmittelbar an das Therapieziel und/oder an die anatomischen Bedingungen des Patienten angepasst zu werden, ohne dass die Orthese vollständig zerlegt oder abgerüstet werden müsste. Bevorzugter Werkstoff der Reklinatorschiene ist Aluminium oder thermoformbarer Kunststoff, besonders ein starrer Thermoplast, der insbesondere mit Carbonfasern mechanisch verstärkt ist. Entsprechende Materialien sind zur Warmanformung aus der Orthopädietechnik und Prothetik bekannt. Zusätzlich sind mechanisch unterschiedlich steife Ausführungen des Reklinatorkerns vorgesehen, die wahlweise, je nach Therapieziel, in die Tasche eingesetzt werden können.

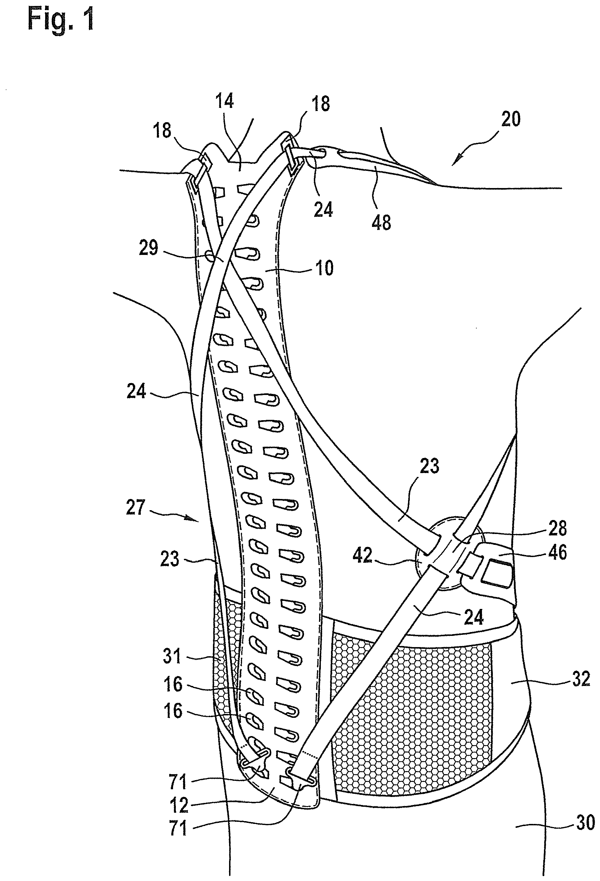

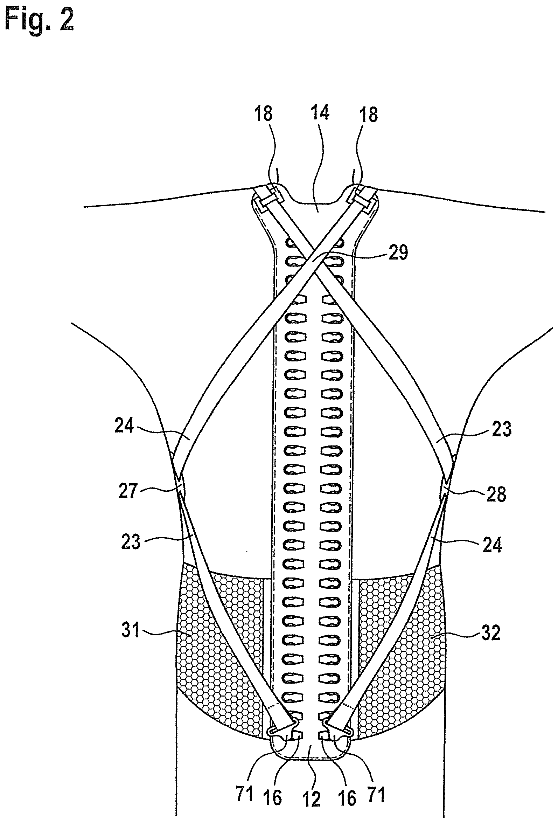

Die Erfindung wird nun durch die in den Figuren dargestellten Ausführungsbeispiele näher beschrieben, ohne dass diese im Einzelnen beschränkend zu verstehen wären. Einzelne in den Figuren abgebildete Erfindungsmerkmale können auch für sich und ohne alle ebenfalls dargestellten optionalen Merkmale Teil einer erfindungsgemäßen Ausführung sein. Figur 1 zeigt eine schräge Rückenansicht einer erfindungsgemäßen Reklinationsorthese, angelegt an einem Patienten. An einem Beckengurt 30 mit den beiden um das Becken herum zugfest verschließbaren Bandagenenden 31, 32 ist mit einer Reklinatorschiene 10 fest verbunden. Die Reklinatorschiene 10 besteht aus einem Reklinatorkern innerhalb einer gefensterten Tasche. Im caudalen Abschnitt 12 der Reklinatorschiene sind in Verankerungspunkten 16 Ankerösen 71 der Gurte 23, 24 eingehängt. Die Gurte 23, 24 verlaufen von diesen Verankerungspunkten 16 im caudalen Abschnitt 12 über den Beckenkamm an der Flanke des Patienten nach vorne und bilden dort jeweils einen Schultergurtabschnitt 20, wo sie über den vorderen Schulterabschnitt zurück nach dorsal über Führungen 18 am cranialen Abschnitt 14 der Reklinatorschiene 10 verlaufen und in einem dorsalen zentralen Kreuzungspunkt 29 kreuzen, etwa mittig und im Bereich der oberen Brustwirbel des Patienten. Die Gurte 23, 24 verlaufen dann auf der gegenüberliegenden Körperseite und kreuzen dann in den lateralen thorakalen Kreuzungspunkten 27, 28 und enden im ventralen cranialen Gurtenden 25, 26 (in Figur 1 nicht gezeigt), die an einem ventralen Abschnitt des Beckengurts 30 unter Spannung festlegbar sind. An den lateralen thorakalen Kreuzungspunkten 27, 28 sind die dort kreuzenden Gurte 23, 24 in Kreuzungsführungen 42 geführt, welche auf der zum Körper hin gerichteten Seite mit Polsterungen versehen sind. Zusätzlich tragen die Gurte 23, 24 an den Hauptpunkten der Krafteinleitung/Druckeinleitung bevorzugt verschiebliche Polsterungen und/oder Pelotten: eine Polsterung 48 im Bereich der Schulter, eine Polsterung 46 im Bereich des Beckenkamms. Figur 2 zeigt eine frontale Rückenansicht der Situation nach Figur 1.

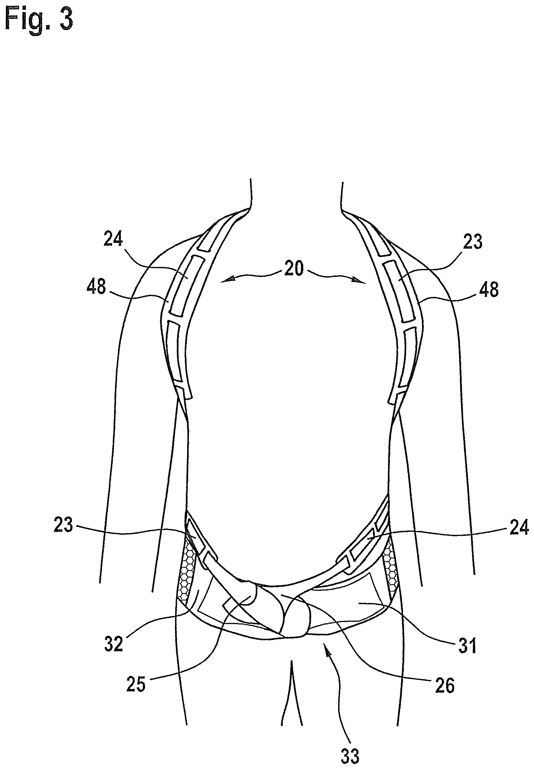

Figur 3 zeigt die Frontalansicht des Zustands nach Figur 1 und 2. Die beiden Bandagenenden 31, 32 des Beckengurts 30 sind durch Übereinanderlegen von Klettelementen unter Spannung miteinander verbunden. Die Gurte bilden im Bereich der Schulter jeweils Schultergurtabschnitte 20, an denen die hauptsächliche Krafteinleitung der Gurte in den Körper erfolgt. In dieser dargestellten Ausführung werden die beiden Gurtenden 24, 25 der Gurte 23, 24 jeweils dort am ventralen Abschnitt des Beckengurts 33, besonders auf einem der beiden Bandagenenden 31, 32 ebenfalls bevorzugt mittels Klettverbindung festgelegt. Der Beckengurt 30 sitzt auf dem Beckenkamm und Becken des Patienten auf, und zwar deutlich unterhalb des Bauchnabels. Die Bandagenenden 31, 32 des Beckengurts werden im Bereich des Unterbauchs oder Schambeins des Patienten geschlossen.

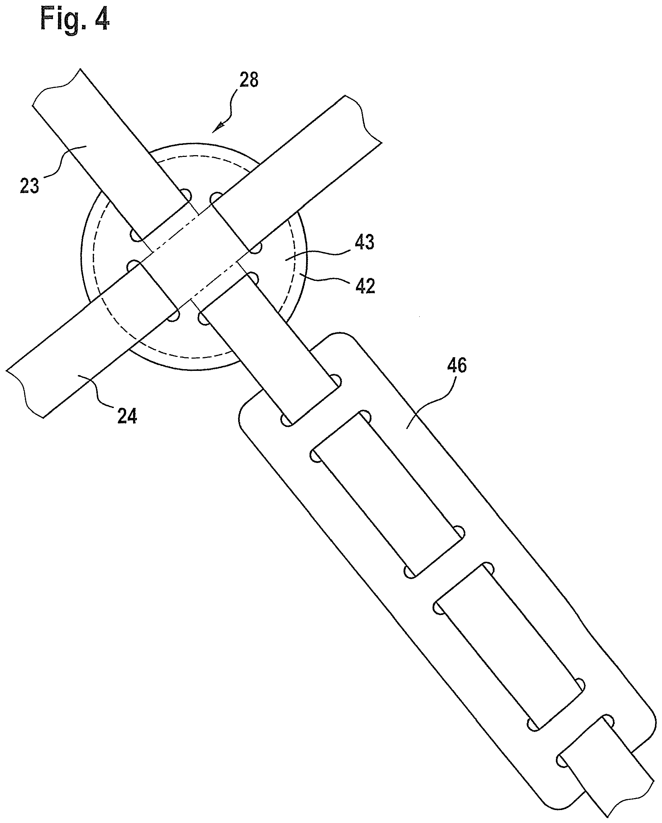

Figur 4 zeigt eine Detailansicht der optionalen Kreuzungsführungen 42 mit darunter liegender Polsterung 43 an dem lateralen thorakalen Kreuzungspunkt 28 der Gurte 23, 24. Zusätzlich ist ein Polsterelement 46 an dem Gurt 23 vorhanden, welches im Bereich des vorderen Beckenkamms positionierbar ist, und dort einen zusätzlichen Druckeinleitungspunkt bildet.

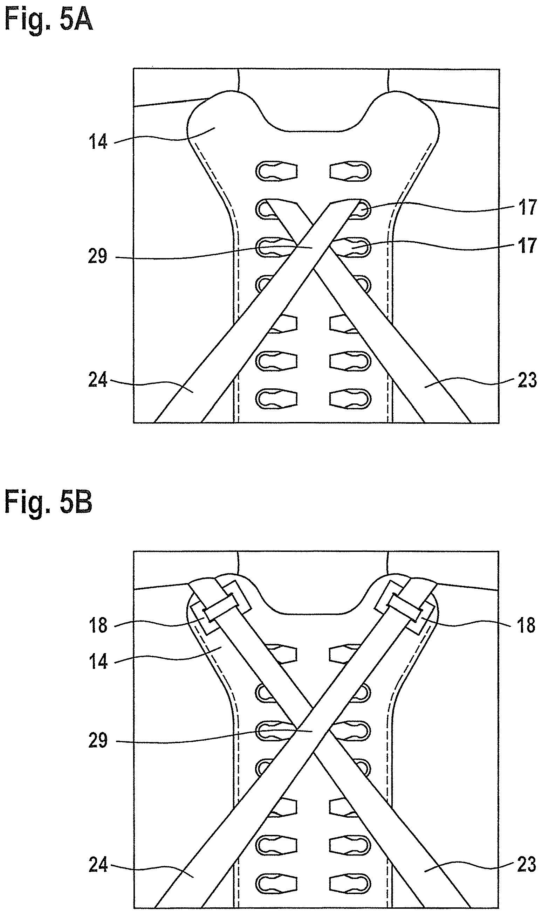

Figuren 5A und 5B zeigen verschiedene Ausgestaltungen der Gurtführungen der Schultergurte 23, 24 im Bereich des cranialen Abschnitts 14 der Reklinatorschiene. Figur 5A zeigt eine Variante, wo die Gurte durch Ausnehmungen in der Reklinatorschiene geführt werden und dort bevorzugt frei laufen. Es können mehrere voneinander beabstandete Durchführungen 17 an der Reklinatorschiene vorgesehen sein, um die Position der Gurtführung anzupassen. Eine alternative und bevorzugte Ausgestaltung zeigt Figur 5B, wo separate Führungsösen 18 die Gurte führen. Die Führungsösen können positionsvariabel an der Reklinatorschiene 10 festgelegt sein.

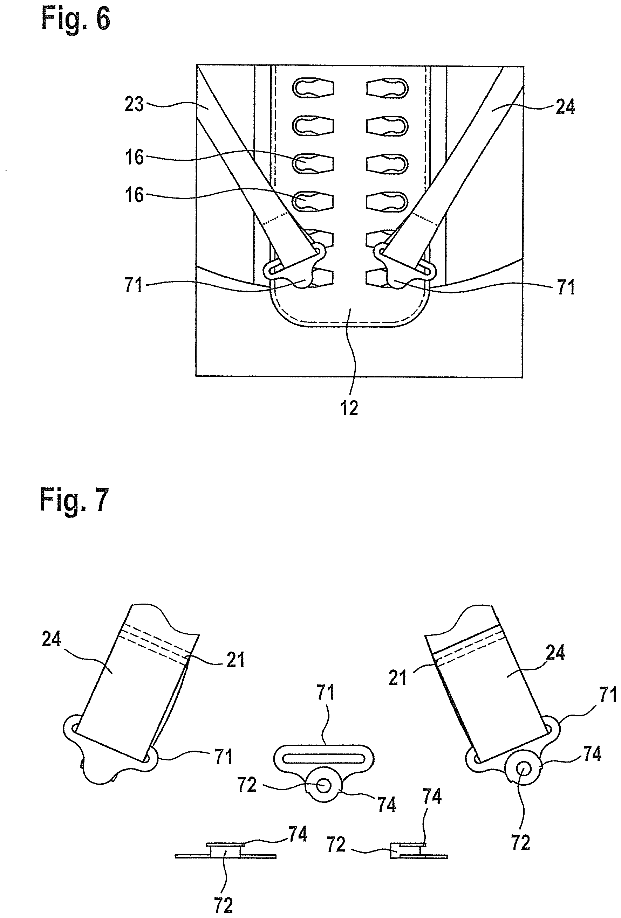

Figur 6 zeigt eine Detailansicht der Verankerung der Gurte 24 oder 23 am caudalen Abschnitt 12 der Reklinatorschiene mittels Ankerösen 71, worin jeweils die Gurte 24 oder 23 geführt und dort fixiert sind. Wobei die Ankerösen 71 in an der Reklinatorschiene ausgebildeten Verankerungspunkten 16 variabel festlegbar sind. Dazu zeigt Figur 7 Detailansichten der Ankerösen 71 in Draufsichten und Schnittansichten. Die Ankerösen besitzen in dieser Ausgestaltung eine Rastnase 72 mit Hinterschnitt und Schulter 74, welche spezifisch ausgebildet sind, um in entsprechend ausgebildete Verankerungspunkte 16 der Reklinatorschiene 10 einzurasten und nach Verdrehen in den entsprechenden Arbeitswinkel aufgrund der Schulter 74 dort gegen Herausfallen geschützt werden, aber um die Rastnase 72 verschwenkbar sind. In der dargestellten Ausführung sind die Gurtenden dort, nachdem sie durch die Ankerösen 71 geführt sind, an Verbindungsflächen 21 fest vernäht oder mit sich selbst verklettet oder verknöpft oder verhakt.

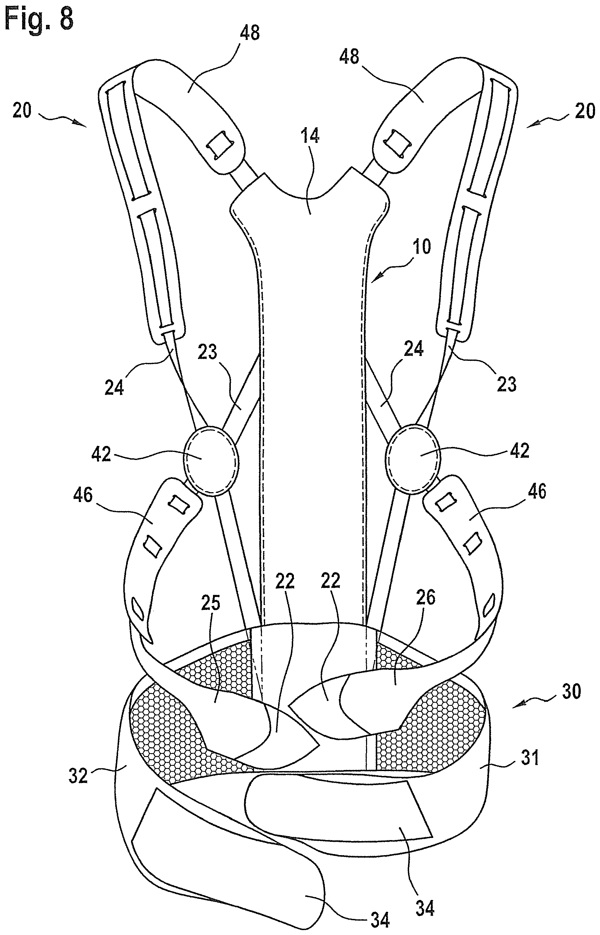

Schließlich zeigt Figur 8 eine Gesamtansicht der erfindungsgemäßen Reklinationsorthese gemäß den vorstehenden Figuren 1 bis 7. Die Gurtenden 25, 26 sind bevorzugt zusätzlich mit Eingreiftaschen 22 versehen, womit der Patient die Gurtenden 25, 26 zum Spannen besser ergreifen kann. Erkennbar sind auch die Flauschzonen 34 der beiden Bandagenenden 31 und 32, die zu deren gegenseitiger Verklettung einerseits und zur Aufnahme der mit Klettelementen versehenen Gurtenden 25, 26 andererseits vorgesehen sind.

## Patentansprüche

1. Reklinationsorthese zum Anlegen an einen Patienten enthaltend einen Beckengurt (30), eine starre Reklinatorschiene (10) und zwei einzelne spannbare Schultergurte (23,24), **dadurch gekennzeichnet, dass**, wenn die Reklinationsorthese an den Patienten angelegt ist, die Schultergurte (23,24) jeweils von einem caudalen Abschnitt (12) der Reklinatorschiene (10) aufsteigend jeweils einen ipsilateralen Schultergurtabschnitt (20) bilden und von dort zurück über Führungsösen (18) an einem cranialen Abschnitt (14) der Reklinatorschiene (10) verlaufen, wo sie sich in einem dorsalen zentralen Kreuzungspunkt (29) kreuzen und von dort kontralateral absteigend verlaufen, wo sie sich in lateralen thorakalen Kreuzungspunkten (27,28) mit dem jeweils aufsteigenden ipsilateralen Gurt kreuzen, und dann in ventralen Gurtenden (25,26), die an einem ventralen Abschnitt (33) des Beckengurts (30) unter Spannung festlegbar sind, auslaufen.

2. Reklinationsorthese nach Anspruch 1, wobei sich ipsilateraler und kontralateraler Gurt (23,24) jeweils in den beiden lateralen thorakalen Kreuzungspunkten (27,28) in einem etwa rechten Winkel kreuzen.

3. Reklinationsorthese nach Anspruch 1 oder 2, wobei die beiden Gurte (23,24) an den drei Kreuzungspunkten (27,28,29) und in den Führungsösen (18) jeweils frei laufen und ausschließlich an dem caudalen Abschnitt (12) der Reklinatorschiene (10) fixiert und an ihren Gurtenden (25,26) an dem ventralen Beckengurtabschnitt (33) festlegbar sind.

4. Reklinationsorthese nach einem der der Ansprüche 1 bis 3, wobei die Gurte (23,24) in den beiden lateralen thorakalen Kreuzungspunkten (27,28) in Kreuzungsführungen (42) frei laufend geführt sind.

5. Reklinationsorthese nach einem der Ansprüche 1 bis 3, wobei die Gurte (23,24) in den beiden lateralen thorakalen Kreuzungspunkten (27,28) in Kreuzungsführungen (42) geführt und dort festlegbar sind.

6. Reklinationsorthese nach einem der vorstehenden Ansprüche, wobei der caudale Abschnitt (12) der Reklinatorschiene (10) in der Höhe des Ileosakral-Gelenks positionierbar ist und der craniale Abschnitt (14) der Reklinatorschiene (10) an den oberen Brustwirbeln oder Halswirbeln positionierbar ist.

7. Reklinationsorthese nach einem der vorstehenden Ansprüche, wobei die Gurte (23,24) an ihrem Ausgangspunkt an dem caudalen Abschnitt (12) der Reklinatorschiene (10) jeweils durch Ankerösen (71) geführt und dort fixiert sind, wobei die Ankerösen (71) in an der Reklinatorschiene (10) ausgebildeten Verankerungspunkten (16) festgelegt sind.

8. Reklinationsorthese nach Anspruch 7, wobei die Reklinationsschiene (10) zumindest in ihrem caudalen Abschnitt (12) mehrere Verankerungspunkte (16) aufweist, worin die Gurte (23,24) mittels Ankerösen (71) jeweils positionsvariabel zur Anpassung der Reklinationswirkung festlegbar sind.

9. Reklinationsorthese nach einem der vorstehenden Ansprüche, wobei der Beckengurt (30) auf Becken und Beckenkamm, und zwar unterhalb des Bauches, positionierbar und dort unter Spannung festlegbar ist.

10. Reklinationsorthese nach einem der vorstehenden Ansprüche, wobei die Stützkraft zur Stützung der Brust- und Lendenwirbelsäule nach Anlegen des Beckengurts (30) auf Becken und Beckenkamm vollständig erzeugbar ist durch Ziehen der beiden Gurtenden (25,26) der beiden einzelnen frei laufenden Gurte (23,24) und Festlegen unter Spannung an dem ventralen Beckengurtabschnitt (33).

## Claims

1. Reclination orthosis for fastening on a patient, including a pelvic strap (30), a rigid reclinator splint (10), and two individual tensible shoulder straps (23, 24), **characterized in that** when the reclination orthosis is fastened on a patient the shoulder straps (23, 24) each form an ipsilateral shoulder strap section (20), respectively, running upward from a caudal section (12) of the reclinator splint (10), from where they run back via guide grommets (18) to a cranial section (14) of the reclinator splint (10), where they cross each other in a dorsal central crossing point (29) and contralaterally run downward from there, where they cross in lateral thoracic crossing points (27, 28) with the respectively upward running ipsilateral strap, and then end in ventral strap ends (25, 26), which are affixable in tension at a ventral section (33) of the pelvic strap (30).

2. Reclination orthosis according to claim 1, wherein ipsilateral and contralateral strap (23, 24) cross at the two lateral thoracic crossing points (27, 28) at roughly a square angle, respectively.

3. Reclination orthosis according to claim 1 or 2, wherein both straps (23, 24) run freely at the three crossing points (27, 28, 29) and in the guide grommets (18), respectively, and are exclusively fixated at the caudal section (12) of the reclinator splint (10), and are affixable at their strap ends (25, 26) at the ventral pelvic strap section (33).

4. Reclination orthosis according to any one of claims 1 to 3, wherein the straps (23, 24) are guided in both lateral thoracic crossing points (27, 28) in a free running manner in crossing guides (42).

5. Reclination orthosis according to any one of claims 1 to 3, wherein the straps (23, 24) are guided in both lateral thoracic crossing points (27, 28) in crossing guides (42) and are affixable therein.

6. Reclination orthosis according to any one of the preceding claims, wherein the caudal section (12) of the reclinator splint (10) can be positioned at the level of the ileosacral joint, and the cranial section (14) of the reclinator splint (10) can be positioned at the upper thoracic vertebrae or cervical vertebrae.

7. Reclination orthosis according to any one of the preceding claims, wherein the straps (23, 24) are each guided at their starting point at the caudal section (12) of the reclinator splint (10) through anchor grommets (71), where they are affixed, and wherein the anchor grommets (71) are fixated at anchoring points (16) formed at the reclinator splint (10).

8. Reclination orthosis according to claim 7, wherein the reclination splint (10) has several anchoring points (16) at least in its caudal section (12), wherein the straps (23, 24) are each affixable by means of anchor grommets (71), at variable positions for adaption of the reclination effect.

9. Reclination orthosis according to any one of the preceding claims, wherein the pelvic strap (30) can be positioned on the pelvis and iliac crest below the belly, where it can be affixed under tension.

10. Reclination orthosis according to any one of the preceding claims, wherein the supporting force to support the thoracic and lumbar spine after applying the pelvic strap (30) on the pelvis and iliac crest can be fully generated by pulling both strap ends (25, 26) of both individual free running straps (23, 24) and affixing them under tension at the ventral pelvic strap section (33).

## Revendications

1. Orthèse de réclination pour application à un patient, incluant une ceinture de bassin (30), un rail réclinateur rigide (10) et deux sangles d'épaule (23, 24) individuelles pouvant être tendues, **caractérisée en ce que**, lorsque l'orthèse de réclination est appliquée au patient, les sangles d'épaule (23, 24) forment respectivement, en montant depuis une section caudale (12) du rail réclinateur (10), une section de bretelle ipsilatérale respective (20) et, de là, s'étendent en retour par le biais d'œillets de guidage (18) sur une section crânienne (14) du rail réclinateur (10), où elles se croisent en un point de croisement central dorsal (29) et de là s'étendent en descendant de manière contralatérale, où elles se croisent en des points de croisement latéraux thoraciques (27, 28) avec la sangle ipsilatérale respectivement montante, et se terminent ensuite en extrémités ventrales de sangles (25, 26), lesquelles peuvent être immobilisées sous tension au niveau d'une section ventrale (33) de la ceinture de bassin (30).

2. Orthèse de réclination selon la revendication 1, dans laquelle les sangles ipsilatérale et contralatérale (23, 24) se croisent respectivement à angle approximativement droit aux deux points de croisement latéraux thoraciques (27, 28).

3. Orthèse de réclination selon la revendication 1 ou 2, dans laquelle les deux sangles (23, 24) passent librement respectivement sur les trois points de croisement (27, 28, 29) et dans les œillets de guidage (18) et peuvent être fixées exclusivement à la section caudale (12) du rail réclinateur (10) et peuvent être immobilisées par leurs extrémités de sangles (25, 26) au niveau de la section ventrale de ceinture de bassin (33).

4. Orthèse de réclination selon l'une des revendications 1 à 3, dans laquelle les sangles (23, 24) sont guidées en passage libre dans des guidages d'intersection (42) dans les deux points de croisement latéraux thoraciques (27, 28).

5. Orthèse de réclination selon l'une des revendications 1 à 3, dans laquelle les sangles (23, 24) sont guidées dans des guidages d'intersection (42) dans les deux points de croisement latéraux thoraciques (27, 28) et peuvent y être immobilisées.

6. Orthèse de réclination selon l'une des revendications précédentes, dans laquelle la section caudale (12) du rail réclinateur (10) est positionnable dans la hauteur de l'articulation sacro-iliaque et la section crânienne (14) du rail réclinateur (10) est positionnable sur les vertèbres thoraciques supérieures ou les vertèbres cervicales.

7. Orthèse de réclination selon l'une des revendications précédentes, dans laquelle les sangles (23, 24) sont guidées respectivement par des œillets d'ancrage (71) à leur point de sortie sur la section caudale (12) du rail réclinateur (10) et y sont fixés, dans laquelle les œillets d'ancrage (71) sont immobilisés dans des points d'ancrage (16) réalisés au niveau du rail réclinateur (10).

8. Orthèse de réclination selon la revendication 7, dans laquelle le rail réclinateur (10) comporte plusieurs points d'ancrage (16) au moins dans sa section caudale (12), dans lesquels les sangles pouvant être immobilisées de manière respectivement variable en position pour adapter l'effet d'réclinaison au moyen d'œillets d'ancrage (71).

9. Orthèse de réclination selon l'une des revendications précédentes, dans laquelle la ceinture de bassin (30) peut être positionnée sur le pelvis et la crête iliaque, à savoir en-dessous du ventre, et peut être y être immobilisée sous tension.

10. Orthèse de réclination selon l'une des revendications précédentes, dans laquelle la force de soutien permettant de soutenir la colonne vertébrale thoracique et lombaire peut être complètement obtenue, après application de la ceinture de bassin sur le pelvis et la crête iliaque, par traction des deux extrémités de sangles (25, 26) des deux sangles individuelles libres (23, 24) et immobilisation sous tension au niveau de la section ventrale de ceinture de bassin (33).
